# EUROPEAN PATENT APPLICATION

(11) **EP 4 101 458 A1**
(43) Date of publication of application: **14.12.2022**
(21) Application number: 22178110.7
(22) Date of filing: 09.06.2022
(51) Int. Cl.: A61K 36/28, A61K 33/30, A61K 33/06, A61K 33/04, A61K 31/11, A61K 31/51, A61K 31/4415, A61K 31/714, A61K 31/375, A61K 31/355, A61P 1/16, A61P 1/14, A61P 9/00, A61P 25/00, A61K 31/00, A61K 33/00

(54) **PHYTOTHERAPY COMPOSITION ASSOCIATED TO POLYVITAMIN AND MINERAL SUPPLEMENTS**

(30) Priority: 09.06.2021 BR 102021011150
(71) Applicant: Campos Barbosa, Fernando, 04119-060 São Paulo (BR); Pereira Rezende, Janaina Drawanz, 04191-190 São Paulo (BR)
(72) Inventor: Campos Barbosa, Fernando, 04119-060 São Paulo (BR); Pereira Rezende, Janaina Drawanz, 04191-190 São Paulo (BR)
(74) Representative: Torner, Juncosa I Associats, SL

(57) **Abstract**

This invention is related to a phytotherapic composition associated to polyvitamin and mineral supplements for anti-hepatotoxic and antioxidant treatment, besides helping hepatic protein synthesis and the circulatory system and contributing directly to neurotransmitter synthesis, whereby its hepatoprotective action helps patients with liver diseases or developing hepatobiliary disorders, providing relief in dyspepsia and liver-related digestive problems.

## Description

### Field of the invention

This invention relates to a phytotherapic composition associated to polyvitamin and mineral supplements in anti-hepatotoxic, antioxidant treatment, in addition to helping hepatic protein synthesis and the circulatory system, and contributing directly to the neurotransmitter synthesis, whereby the hepatoprotective action helps people living with liver diseases or for the development of hepatobiliary disorders, providing relief in dyspepsia and liver-related digestive problems.

### Background to the invention

There are currently no phytotherapic products associated to polyvitamin and mineral supplements. Thus, the inventors of this invention have ascertained the need to develop a phytotherapic composition associated to polyvitamin and mineral supplements in order to enable synergic action between the substances, aimed particularly at health maintenance and for aiding and supplementing treatments. The presence of these bioactive components encourages biological and physiological actions that promote and maintain the health of the human organism.

This phytotherapic composition associated to polyvitamins and minerals is safe and easy to access for the population, obtained with no need for a medical prescription. However, it increases the therapeutic arsenal of medical practitioners, supplementing their prescriptions.

This new proposed solution works with the underlying metabolic condition to maintain health with quality of life, such as the liver function.

The advantages of this composition consist of functional synergism in the action of its bioactive compounds, promoting anti-hepatotoxic, antioxidant actions while fostering hepatic protein synthesis, helping the circulatory system and contributing directly to the neurotransmitter synthesis.

### Brief description of this invention

This invention provides a phytotherapic composition associated to polyvitamin and mineral supplements comprising (i) *Silybum marianum* (L); (ii) choline; (iii) a vitamin complex, comprising vitamin A, vitamin B6, vitamin C, vitamin D, vitamin E, vitamin B1, and vitamin B12; (iv) selenium; (v) zinc; and (vi) magnesium.

The biological and pharmacological properties of *Silybum marianum* (L.), or milk thistle, are attributed to a flavonolignan complex known as silymarin, which was the first compound isolated from its fruits in 1968.

The silymarin mixture is composed predominantly of silybin (30% -50%) which may vary in percentages of isosilybin, silycristin and silydianin. In addition to silymarin and other flavonolignans, 20% -30% of the fruit is composed of fatty acids; 25% - 30% protein; 0.038% tocopherol; 0.63% sterols, and some compounds such as deoxyflavonolignans.

Milk thistle is used for multiple medicinal purposes. Several components exhibit various mechanisms of action that may be hepatoprotective, including antiinflammatory, antioxidant, toxin cleanser, protein synthesis inducer, and fibrosis treatment activities.

As mentioned, the main action of the active ingredients of *Silybum marianum* is protecting the liver. Among the pharmacological applications used for this purpose, hepatoprotective action in alcoholic liver disease, viral hepatitis, cirrhosis, drug toxicity, mushroom poisoning, diabetes, patients with chronic liver disease, and hypocholesterolemic actions are mentioned in the literature.

Milk thistle is a traditional phytotherapic product, used primarily to relieve dyspepsia and liver-related digestive complaints. For this purpose, doses ranging from 300 mg - 600 mg, may be taken two to three times a day, with doses of up to 1800 mg/day of the fruit powder being well accepted, with well-established traditional use.

Contraindications include hypersensitivity to the active substances and plants in the Asteraceae (Compositae) family. Its use by children and adolescents under 18 years of age has not been established, due to the lack of adequate data.

Known adverse effects include mild gastrointestinal symptoms such as dry mouth, nausea, gastric irritation, and diarrhea; headache; allergic reactions (dermatitis, urticaria, rash, pruritus, anaphylaxis, asthma) may occur. The frequency is not known.

In addition to using milk thistle to maintain health and as a treatment aid and supplement, a purpose of this invention is to use certain vitamins and minerals for the same purpose, which help maintain liver functions.

Appropriate vitamin supplementation is necessary to maintain normal cell function and renewal, in addition to promoting tissue repair. There is growing evidence that specific vitamin requirements increase when under stress, due to greater loss or higher use. Furthermore, several vitamins play important roles in regulating immune responses and promoting tissue healing.

Thus, this concept proposes a phytotherapic composition associated to polyvitamin and mineral supplements that comprises the components listed below.

Choline is hepatoprotective and lipotropic, preventing the occurrence of hepatic steatosis. Low levels of choline contribute to the pathophysiology of liver diseases by disrupting mitochondrial bioenergetics and affecting fatty acid beta oxidation.

Vitamins A, C, D, And, and B6 have potential immune function effects. Vitamins A and C have effects on healing, and vitamins A, C, and E offer defense against free radical damage, with high demands in patients with liver disease and pancreatitis.

Vitamin A deficiency is related to a decrease in gastrointestinal absorption and hepatic mobilization. It may also cause dermatitis, night blindness or photophobia, with higher risk of neoplastic disorders such as hepatocellular carcinoma.

Vitamin B6 acts as a coenzyme in more than 100 metabolic reactions involving amino acid metabolism. Inflammation, reduced intake, and altered vitamin B6 metabolism seen in patients with liver changes may lead to low plasma levels, resulting in seborrheic dermatitis, seizures, stomatitis, glossitis, depression, confusion, and microcytic anemia.

Vitamin C is important for the formation of collagen, carnitine, and catecholaminergic hormones, in addition to its already known antioxidant properties. Risk factors for deficiency include alcohol abuse, poor quality diet, physiological stress, and dialysis, the symptoms of which are characterized by perifollicular petechiae, keratosis, ecchymosis, slow healing, glossitis, anemia, and fatigue.

For vitamin D, there is evidence that very low plasma levels are related to increased mortality in patients with chronic liver disease. Enteropathic hypertension and cholestasis may be involved in vitamin D deficiency by altering absorption. Vitamin D is oxidized in the liver for calcidiol synthesis, so alterations to the liver parenchyma or obstructive diseases may reduce this metabolite.

In addition to its antioxidant and anti-free radical actions, Vitamin E prevents the formation of fibrosis. Changes arising from hepatic cholestatic diseases may lead to reduced absorption and deficiency, characterized by clumping platelets, hemolytic anemia, neurological symptoms, skeletal myopathy, and pigmentary retinopathy.

Thiamine (B1) is required for carbohydrate and lipid metabolism, which occurs mainly in the liver. A lack of this vitamin is common and not unique to alcoholic diseases. A reduction in intake and absorption, as well as depletion of liver reserves, are factors related to this deficiency. Malnutrition or alcoholism can lead to a thiamine deficiency - a condition called *beriberi,* characterized by cardiovascular symptoms (stiffness and muscle cramps, edema of the face and extremities, anorexia, mental confusion, ophthalmoplegia, and ataxia) and gastrointestinal symptoms (indigestion, constipation, gastric atony, deficiency of hydrochloric acid secretion), irritability and depression.

Alcoholics may develop Wernicke's encephalopathy, with symptoms such as mental confusion, ophthalmoplegia, ataxia, and Korsakoff's psychosis, a condition with progressive and irreversible brain damage. In the latter, amnesia for recent events occurs, with memory gaps for events that occurred months before the thiamine deficiency. Thiamine concentration may drop in the presence of a high carbohydrate diet.

Taking certain medications and drinking alcohol reduces vitamin B12 absorption. Patients with malabsorption syndrome and gastrointestinal disorders are candidates for developing shortages of this vitamin. Symptoms of the lack of this vitamin are megaloblastic anemia accompanied by macrocytosis, leukopenia and thrombocytopenia, anorexia, constipation, glossitis, neurological changes such as peripheral paresthesia (hands and feet), memory loss, decreased sense of position, mental confusion, depression, and psychosis.

Selenium is important in the body's antioxidant defense system mechanism, as an essential component of selenoproteins. It decreases in some liver diseases, and may be a side effect of a low immune response due to selenium deficiency.

Zinc has an important action, comprising approximately 120 enzymes (carbonic anhydrase, carboxypeptidase, alkaline phosphatase, oxidoreductases, transferases, ligases, hydrolases, lyases, and isomerases), and is also a key element for DNA polymerase, reverse transcriptase, RNA polymerase, tRNA synthetase, and the protein chain elongation factor.

Zinc deficiency has a pronounced effect on nucleic acid metabolism and influences protein and amino acid metabolism. The mechanism involves changes in the nature of RNA polymerase and mRNA composition, with consequences on histone synthesis. Zinc deficiency is common in liver diseases, where altered amino acid and protein metabolism, reduced albumin synthesis, oxidative stress, and hepatic shunts are observed. The physical, neurological, and clinical manifestations of zinc deficiency may include skin lesions such as angular cheilosis, reduced wound healing, night blindness due to secondary vitamin A deficiency, altered taste and smell, altered mental status, insulin resistance, and iron overload. Zinc deficiency may also damage liver cells and tissues, with negative impacts on its ability to regenerate.

Magnesium deficiency may cause peripheral vasodilation, cardiac arrhythmia, and seizures, with chronic alcoholism, malabsorption syndrome, and increased loss through the gastrointestinal tract among the main causes. Alcohol consumption may also cause folate and magnesium deficiency. Alcohol has been shown to impair magnesium transport and homeostasis in the brain, skeletal muscle, and heart, and consequently the liver. Magnesium is a necessary cofactor for thiamine pyrophosphate metabolism, and should be monitored in cases of thiamine deficiency. It is also involved in the occurrence of mast cell-dependent liver fibrosis and steatosis.

### Detailed description of this invention

This invention describes a phytotherapic composition associated to polyvitamin and mineral supplements comprising (i) *Silybum marianum* (L); (ii) choline; (iii) a vitamin complex, comprising vitamin A, vitamin B6, vitamin C, vitamin D, vitamin E, vitamin B1 and vitamin B12; (iv) selenium; (v) zinc; and (vi) magnesium, wherein: (i) *Silybum marianum* (L) is present in the range of 16% - 20% p.p; (ii) choline is present in the range of 19% - 23% p.p; (iii) vitamin A is present in the range of 0.25% - 0.65% p.p., vitamin B6 is present in the range of 0.19% - 0.23%, vitamin C is present in the range of 41% - 45%, vitamin D is present in the range of 0.001% - 0.003% p.p., vitamin E is present in the range of 11% - 15% p.p., vitamin B1 is present in the range of 0.11% - 0.15% p.p., and vitamin B12 is present in the range of 0.0002% - 0.0004% p.p.; (iv) selenium is present in the range of 0.011% - 0.015% p.p.; (v) zinc is present in the range of 1% - 3% p.p.; and (vi) magnesium is present in the range of 0.009%-0.013% p.p., with the range based on 100% of the total weight of the composition.

Preferably, this invention describes a phytotherapic composition associated to polyvitamin and mineral supplements comprising (i) 18.5% p.p. *Silybum marianum* (L); (ii) 21.7% p.p. choline; (iii) vitamin complex wherein 0.45% p.p. is vitamin A, 0.21% p.p. is vitamin B6, 43.7% p.p. is vitamin C, 0.002% p.p. is vitamin D, 13% p.p. is vitamin E, 0.13% p.p. is vitamin B1; 0.0003% p.p. is vitamin B12; (iv) 0.013% p.p. is selenium; (v) 2.4% p.p. is zinc; and (vi) 0.01% p.p. is magnesium, with the range based on 100% of the total weight of the composition.

The phytotherapic composition associated with the polyvitamin and mineral supplements addressed by this invention is prepared in powder form, suitable for encapsulation and oral administration.

This invention also provides for the use of the phytotherapic composition associated to polyvitamin and mineral supplements as defined above, in the preparation of an anti-hepatotoxic, antioxidant supplement, aiding hepatic protein synthesis and the circulatory system and contributing directly to neurotransmitter synthesis, providing relief in dyspepsia and liver-related digestive problems to patients with liver diseases or the development of hepatobiliary disorders.

## Claims

1. PHYTOTHERAPIC COMPOSITION ASSOCIATED TO POLYVITAMINS AND MINERAL SUPPLEMENTS, **characterized in that** it comprises: (i) *Silybum marianum* (L); (ii) choline; (iii) a vitamin complex, comprising vitamin A, vitamin B6, vitamin C, vitamin D, vitamin E, vitamin B1 and vitamin B12; (iv) selenium; (v) zinc; and (vi) magnesium, whereby: (i) *Silybum marianum* (L) is present in the range of 16% - 20% p. p; (ii) choline is present in the range of 19% - 23% p.p; (iii) vitamin A is present in the range of 0.25% - 0.65% p.p., vitamin B6 is present in the range of 0.19% - 0.23% p.p., vitamin C is present in the range 41% - 45% p.p., vitamin D is present in the range of 0.001% - 0.003% p.p., vitamin E is present in the range of 11% - 15% p.p., vitamin B1 is present in the range of 0.11% - 0.15% p.p. and vitamin B12 is present in the range of 0.0002% - 0.0004% p.p.; (iv) selenium is present in the range of 0.011% - 0.015% p.p.; (v) zinc is present in the range of 1% - 3% p.p.; and (vi) magnesium is present in the range of 0.009% - 0.013% p.p., where the range is based on 100% of the total weight of the composition.

2. PHYTOTHERAPIC COMPOSITION, according to claim 1, **characterized in that** it comprises: (i) 18.5% p.p. *Silybum marianum* (L); (ii) 21.7% p.p. choline; (iii) vitamin complex wherein 0.45% p.p. is vitamin A, 0.21% p.p. is vitamin B6, 43.7% p.p. is vitamin C, 0.002% p.p. is vitamin D, 13% p.p. is vitamin E, 0.13% p.p. is vitamin B1; 0.0003% p.p. is vitamin B12; (iv) 0.013% p.p. is selenium; (v) 2.4% p.p. is zinc; and (vi) 0.01% p.p. is magnesium, where the range is based on 100% of the total weight of the composition.

3. PHYTOTHERAPIC COMPOSITION, according to claims 1 and 2, **characterized in that** it is prepared in powder form, suitable for encapsulation and administration.

4. USE OF A PHYTOTHERAPIC COMPOSITION ASSOCIATED TO POLYVITAMINS AND MINERAL SUPPLEMENTS as defined by one of claims 1 to 3, **characterized in that** it is used in the preparation of an anti-hepatotoxic, antioxidant supplement, assisting hepatic protein synthesis and the circulatory system, contributing directly to neurotransmitter synthesis, providing relief from dyspepsia and hepatic digestive problems for patients with liver diseases or developing hepatobiliary disorders.
